(19) Europäisches Patentamt
European Patent Office
Office européen des brevets

(11) Publication number: **0 426 167 A2**

(12) # EUROPEAN PATENT APPLICATION

(21) Application number: 90120935.3

(22) Date of filing: 31.10.90

(51) Int. Cl.5: C07D 407/14, A61K 31/35

(30) Priority: 02.11.89 JP 284774/89

(43) Date of publication of application: 08.05.91 Bulletin 91/19

(84) Designated Contracting States: DE FR GB IT

(71) Applicant: MEIJI SEIKA KAISHA LTD.
4-16 Kyobashi 2-chome
Chuo-ku Tokyo(JP)

(72) Inventor: Sato, Yoshikazu, Meiji Seika Kaisha, Ltd.
Pharmaceutical Research Lab. 760, Morooka-cho
Kohoku-ku, Yokohama-shi, Kanagawa(JP)
Inventor: Ishii, Shigetaka, Meiji Seika Kaisha, Ltd.
Pharmaceutical Research Lab. 760, Morooka-cho
Kohoku-ku, Yokohama-shi, Kanagawa(JP)
Inventor: Kawaharajo, Katsumi, Meiji Seika Kaisha, Ltd.
Pharmaceutical Research Lab. 760, Morooka-cho
Kohoku-ku, Yokohama-shi, Kanagawa(JP)
Inventor: Koyama, Masao, Meiji Seika Kaisha, Ltd.
Pharmaceutical Research Lab. 760, Morooka-cho
Kohoku-ku, Yokohama-shi, Kanagawa(JP)
Inventor: Sezaki, Masaji, Meiji Seika Kaisha, Ltd.
Pharmaceutical Research Lab. 760, Morooka-cho
Kohoku-ku, Yokohama-shi, Kanagawa(JP)
Inventor: Inoye, Shigeharu, Meiji Seika Kaisha, Ltd.
Pharmaceutical Research Lab. 760, Morooka-cho
Kohoku-ku, Yokohama-shi, Kanagawa(JP)

(74) Representative: WILHELMS, KILIAN & PARTNER Patentanwälte
Eduard-Schmid-Strasse 2
W-8000 München 90(DE)

(54) **Antitumor substance, process for preparing the same, and pharmaceutical composition containing the same.**

(57) A compound represented by formula (I):

EP 0 426 167 A2

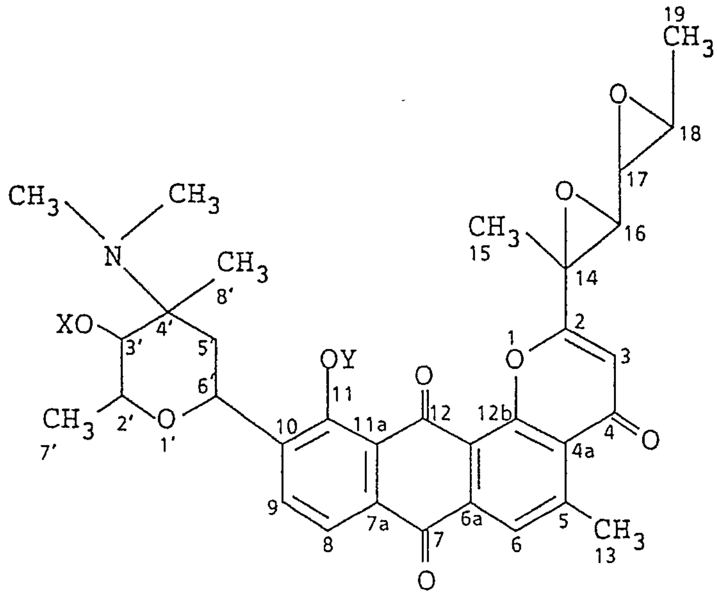

(I)

wherein X and Y each represents a hydrogen atom, a group RCO-, wherein R represents an alkyl group having at least 5 carbon atoms or a substituted or unsubstituted aryl group, or a group $R'SO_2$-, wherein R′ represents a lower alkyl group or a substituted or unsubstituted aryl group, provided that X and Y do not simultaneously represent a hydrogen atom, or a pharmaceutically acceptable salt thereof, which has low acute toxicity and potent antitumor activity.

# ANTITUMOR SUBSTANCE, PROCESS FOR PREPARING THE SAME, AND PHARMACEUTICAL COMPOSITION CONTAINING THE SAME

## FIELD OF THE INVENTION

This invention relates to a novel compound having an antitumor activity, a process for preparing the same, and an anticancer agent containing the same or a pharmaceutically acceptable salt thereof as an active ingredient.

## BACKGROUND OF THE INVENTION

SF2587 substance is an antibiotic isolated from extract of the cultured cells of a strain of actinomycetes belonging to the genus Streptomyces (cf. EP-A-0 326 173), and is represented by formula (II):

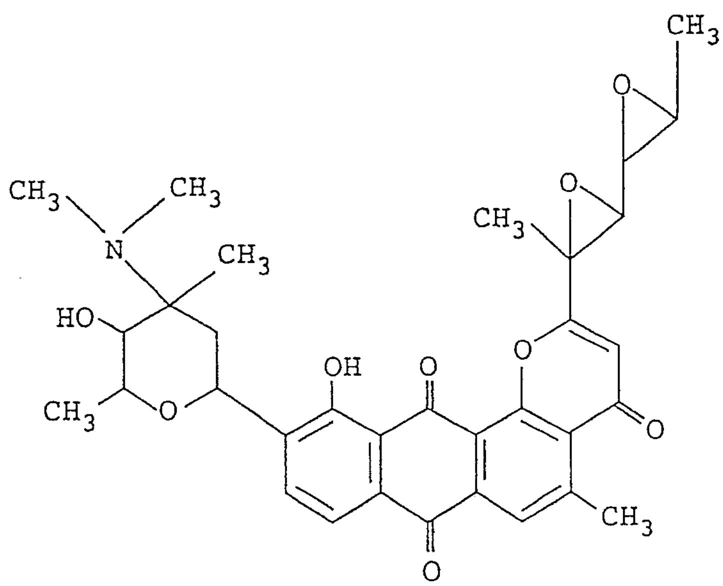

(II)

SF2587 exhibits considerable inhibitory activity on P388 tumor cells and revealed excellent therapeutic, effects in experiments performed on mice transplanted with P388 tumor cells. Nevertheless, since SF2587 substance is of relatively high toxicity ($LD_{50}$: about 0.5-1 mg/kg, mouse, i.p.).

## SUMMARY OF THE INVENTION

An object of the present invention is to provide a devitative of SF2587 substance which has reduced toxicity and enhanced antitumor activity as compared with the SF2587 substance.

The inventors have conducted extensive studies on a method of reducing the toxicity of SF2587 substance while further improving its antitumor activity. As a result, it has now been found that the above object can be attained by an acyl or sulfonyl derivative of SF2587 substance represented by the following formula (I) obtained by chemical conversion of SF2587 substance or a pharmaceutically acceptable salt thereof:

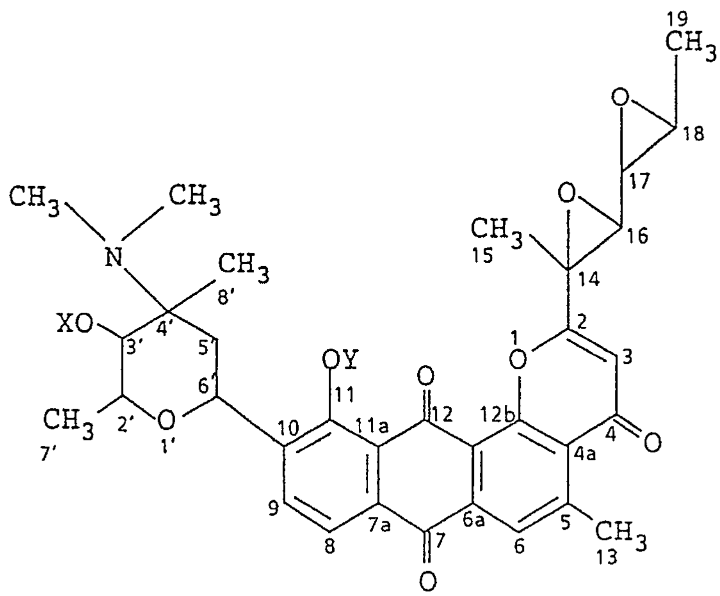

(I)

wherein X and Y each represents a hydrogen atom, a group RCO-, wherein R represents an alkyl group having at least 5 carbon atoms or a substituted or unsubstituted aryl group, or a group $R'SO_2$-, wherein R' represents a lower alkyl group or a substituted or unsubstituted aryl group, provided that X and Y do not simultaneously represent a hydrogen atom.

The present invention further relates to a pharmaceutical composition containing the SF2587 substance derivative represented by formula (I) or a pharmaceutically acceptable salt thereof as an active ingredient in an amount effective for treating cancer.

The present invention furthermore relates to a process for preparing the SF2587 substance derivative represented by formula (I), which comprises reacting the SF2587 substance represented by formula (II):

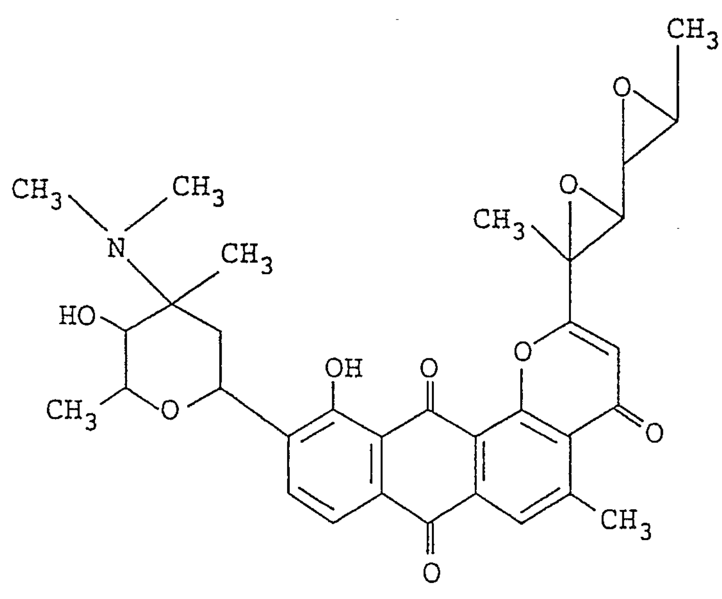

(II),

with an acylating agent or a sulfonylating agent.

## DETAILED DESCRIPTION OF THE INVENTION

In formula (I), the alkyl group represented by R preferably contains 5-19 carbon atoms. The substituted aryl group represented by R include a phenyl, p-hydroxyphenyl, p-tolyl and p-bromophenyl group. Specific examples of the group represented by RCO-includes an n-caproyl, isocaproyl, capryloyl, decanoyl, lauroyl, myristoyl, palmitoyl, stearoyl, benzoyl a p-bromobenzoyl group. Among these, preferred are myristoyl, palmitoyl, stearoyl, benzoyl and decanoyl groups. The lower alkyl group represented by R′ contains 1-5 carbon atoms. The substituted aryl group represented by R′ include a phenyl, p-tolyl, p-nitrophenyl and p-chlorophenyl group. Specific examples of the group represented by $R'SO_2$- include a methanesulfonyl, ethanesulfonyl, p-toluenesulfonyl, p-nitrobenzenesulfonyl and p-chlorobenzenesulfonyl group, with a methanesulfonyl group and a p-toluenesulfonyl group being preferred.

The SF2587 substance can be prepared, for example, by cultivating a microorganism capable of producing the substance and recovering it from the culture.

An example of the SF2587-producing strain is strain SF2587 which has been deposited at the Fermentation Research Institute of Japan under deposit No. BP-2244 in accordance with the Budapest treaty.

The bacteriological characteristics of strain SF2587 are as follows.

I. Morphology

The vegetative mycelium is long-extending and well branched, and is not fragmented under usual conditions. The aerial mycelium is abundant, with good sporulation, on oatmeal agar, inorganic salts-starch agar, yeast extract-malt extract agar, etc. The aerial mycelium is monopodially branched, with no apparent whirl formation. The spore chain at the terminal end of the aerial mycelium is mostly spiral but at times undulating. Electron microscopy reveals that the spore is elipsoidal to cylindrical and measures 0.5 to 0.9 x 0.7 to 1.3 pm. The spore wall ornamentation is smooth. Usually 20 or more spores occur in chains. The sporangium, motile spore, sclerotium, etc. are not observed.

II. Cultural Characteristics

The cultural characteristics of strain SF2587 on various media are shown in Table 1. In the description of color, the color standards given in parentheses are those used in Container Corporation of America's Color Harmony Manual. The observation was made after 14-21 days of incubation at 28°C.

Table 1

| Medium | Growth (Reverse Color) | Aerial Mycerium | Soluble Pigment |
|---|---|---|---|
| Sucrose nitrate agar | Good, topas (3ne) | Abundant, gray (2fe) | None |
| Glucose asparagine agar | Poor-fair, light yellow (2ec) | None | None |
| Glycerol asparagine agar | Fair, colorless | Fair, gray (3fe) | None |
| Calcium malate agar | Fair, gray yellow (2ne) | Abundant, gray (3fe) | None |
| Inorganic salts-starch agar | Good, rose-beige (4ge) | Abundant, gray (3fe) | None |
| Oatmeal agar | Good, rose-beige (4ge) | Abundant, gray (3fe) | None |
| Yeast extract-malt extract agar | Good, light brown (3lg) | Abundant, gray (3fe) | Pale yellow |
| Tyrosine agar | Fair, tan (3ie) | Abundant, Gray (3fe) | None |
| Nutrient agar | Fair, topas (3ne) | Sparse, white | None |
| Bennett agar | Fair,gray yellow (2gc) | Fair, gray (3fe) | None |

III. Physiological Characteristics

(1) Temperature range for growth: On yeast extract-malt extract agar, growth occurs in the temperature range of 15-45°C and good growth at 26-37°C.
(2) Liquefaction of gelatin: Positive

(3) Hydrolysis of starch: Positive
(4) Reduction of nitrate: Negative
(5) Peptonization of skimmed milk: Positive Coagulation of skimmed milk: Positive
(6) Salt resistance: Growth occurs in media containing 10% NaCl but does not in media containing 12% or more of NaCl.
(7) Production of melanoid pigment: Negative

IV. Utilization of Carbon Sources (ISP No. 9 medium)

All of D-glucose, glycerol, D-xylose, L-arabinose, L-rhamnose, D-mannitol,D-fructose, raffinose, myoinositol and sucrose are well assimilated.

V. Cell Wall Composition

As analyzed by the method of Becker et al. (Appl. Microbiol. , 13 , 236 (1965)), the cell wall fraction contains LL-diaminopimellic acid.

Thus, strain SF2587 is considered to belong to the genus Streptomyces , which is among actinomycetes, with aerial mycelium in the Gray color series, the terminal end of aerial mycelium being mostly spiral, a smooth spore surface, and a reverse color of gray yellow with a tinge of red, and does not produce melanoid pigments.

Like other actinomycetes, strain SF2587 is liable to undergo variation in characteristics. For example, mutant strains (spontaneous or induced) as well as transductants and transformants (genetically engineered) of, or derived from, strain SF2587 can also be used for the purposes of this invention only if they are able to produce SF2587 substance. In the method of this invention, the above-mentioned strain is cultivated in a medium containing the ordinary nutrients which microorganisms may utilize. Thus, as nutrient sources, those known sources which have been conventionally utilized in the culture of actinomycetes can be utilized. For example, as carbon sources, use can be made of glucose, glucose or maltose syrup, dextrin, starch, sucrose, molasses, and animal or vegetable oils, preferably maltose syrup, starch, glucose, soybean oil and sucrose. As nitrogen sources, used can be made of soybean meal, wheat germs, corn steep liquor, cottonseed meal, meat extract, peptone, yeast extract, ammonium sulfate, sodium nitrate, urea and so on, preferably soybean meal and Pharamamedia (trade name of cottonseed meal produced by Troders Oil Mill Co., Texas). In addition, it is sometimes advantageous to incorporate various inorganic salts capable of providing sodium, calcium, magnesium, cobalt, chlorine, phosphate, sulfate and other ions. Preferable examples of inorganic salts include $CaCO_3$, $FeSO_4 \cdot 7H_2O$ and $CoCl_2 \cdot 6H_2O$. It is also appropriate to add suitable amounts of organic and/or inorganic substances which assist in the growth of the microorganism and promote production of SF2587 substance. A preferable example thereof is distillers solubles.

The pH value of the medium preferably ranges from 6.0 to 7.5.

To grow the strain, aerobic culture is carried out, and submerged aerobic culture is particularly advantageous. While the incubation temperature may range from 26 to 37° C, it is appropriate to carry out the cultivation at about 28° C in many instances. Though it depends on the medium and cultural conditions used, accumulation of SF2587 substance reaches a peak generally in 2 to 7 days, whether in shake culture of in tank culture (preferably tank culture). When the accumulation of SF2587 substance has become maximal, the incubation is stopped and the desired substance is isolated and purified from the resulting cultured cells.

Since SF2587 substance is fat-soluble, this property can be exploited in its isolation and purification from the culture broth. Thus, there can be advantageously utilized methods of column chromatography using synthetic adsorbents such as Amberlite XAD-2 (Rhom & Haas Co.), Diaion HP-20 (Mitsubishi Kasei Corporation), gel filtration aids such as Sephadex LH-20 (Pharmacia Fine Chemicals), Toyoperal HW-40 (Tosoh Corporation), silica gel, alumina, or solvent extraction processes using ethyl acetate, chloroform and so on.

By any or a suitable combination of these techniques, SF2587 substance can be isolated in high purity.

The compound of formula (I) according to the present invention can be prepared by reacting SF2587 substance with an acylating agent or a sulfonylating agent in the presence of a base. The acylating agent which can be used in this invention includes n-caproic anhydride, n-caproyl chloride, capryloyl chloride, caprylic anhydride, decanoyl chloride, lauroyl chloride, laurylic anhydride, myristoic anhydride, myristoyl chloride, palmitoic anhydride, palmitoyl chloride, stearylic anhydride, stearoyl chloride, benzoic anhydride, benzoyl chloride, and p-bromobenzoyl chloride. Examples of the sulfonylating agent include methanesulfonyl chloride, ethanesulfonyl chloride, p-toluenesulfonyl chloride, p-nitrobenzenesulfonyl chloride and p-chlorobenzenesulfonyl chloride. Both of the acylating reaction and the sulfonylating reaction are carried out at -10° C to 100° C for 12 to 36 hours, preferably at 0 to 50° C for about 24 hours. The acylating agent and the sulfonylating agent are both used in an amount of 2 to 20 moles per mole of SF2587 substance. The base required for these reactions includes pyridine, dimethylaminopyridine and triethylamine. The amount of the base to be used are 100 to 300 moles per mole of SF2587 substance, provided that dimethylaminopyridine is used in a catalytic amount of 0.1 to 0.4 mole per mole of SF2587 substance.

The compound of formula (I) can be purified from the reaction mixture by silica gel column chromatography, followed by recrystallization if required.

Examples of a pharmaceutically acceptable salt of the compound of formula (I) according to the present invention include hydrochloride, sulfate, phosphate, acetate, cit rate, malonate, salicylate, maleate, fumarate, succinate, ascorbate, malate, methanesulfonate, lactate, gluconate, glucronate, amidosulfonate, benzoate and tatrate.

The anticancer composition containing the compound of formula (I) or a pharmaceutically acceptable salt thereof is administered orally or parenterally. When parenterally given, the compound is dissolved in physiological saline or an appropriate buffer solution and formulated into a solution or a suspension for injection, rectal infusion or local application. When orally administered, the compound of formula (I) is mixed with pharmaceutically acceptable vehicles, and the like and, if desired, formulated into gelatin capsules, tablets, etc. each containing 1 to 200 mg of the active ingredient. A suitable dose to mammals inclusive of humans ranges from 0.5 to 20 mg/kg-b.w. one to three times per day.

The present invention is now illustrated in greater detail by way of the following Examples, but it should be understood that the present invention is not construed as being limited thereto.

## EXAMPLE 1

Synthesis of 11-O-Decanoyl SF2587 Substance (Compound 1) and 3′,11-O-didecanoyl SF2587 Substance (Compound 2)

Two milliliters of pyridine were added to 5 mℓ of a dichloromethane solution containing 51 mg of SF2587 substance suspended therein, and 5 mℓ of a dichloromethane solution containing 88 μℓ of decanoyl chloride was further added thereto at room temperature while stirring. To the mixture was further added 5 mg of dimethylaminopyridine, followed by stirring at 25° C for 2 hours. The reaction mixture was poured into 15 mℓ of ice-water and extracted twice with 10 mℓ portions of dichloromethane. The dichloromethane layer was separated, washed twice with 15 mℓ portions of water and then with 15 mℓ of a saturated sodium chloride aqueous solution, and dried over anhydrous sodium sulfate. The sodium sulfate was removed by filtration, and the resulting dichloromethane solution was concentrated under reduced pressure to obtain a yellowish brown oily substance. The substance was dried under reduced pressure overnight and purified by thin-layer chromatography using a silica gel plate and a 5:1 (by volume) mixture of chloroform and methanol as a developing solvent to obtain 47.7 mg of a 11-O-decanoyl derivative of SF2587 substance and 17.0 mg of a 3′,11-O-didecanoyl derivative of SF2587 substance.

Properties of Compound 1: the compound of formula (I) wherein X = H; and Y = $CH_3(CH_2)_8CO$-

FDMS: m/z 743

$$\text{UV } \lambda_{max}^{MeOH} \text{ nm } (\varepsilon): 238\ (32,170),\ 269\ (29,500),\ 352\ (7,620).$$

Properties of Compound 2: the compound of formula (I) wherein X = $CH_3(CH_2)_8CO$-, Y = $CH_3(CH_2)_8CO$-)

FDMS: m/Z 897 ($M^+$)

$$\text{UV } \lambda_{max}^{MeOH} \text{ nm } (\varepsilon): 240\ (37,760),\ 262\ (35,340),\ 361\ (8,520).$$

EXAMPLE 2

Synthesis of 11-O-Stearoyl SF2587 Substance (Compound 3) and 3′,11-O-distearoyl SF2587 Substance (Compound 4)

Four milliliters of pyridine were added to 10 mℓ of a dichloromethane solution containing 200 mg of SF2587 substance suspended therein, and 10 mℓ of a dichloromethane solution containing 546 mg of stearoyl chloride was added thereto at room temperature while stirring. To the mixture was further added 11 mg of dimethylaminopyridine, followed by stirring at 25 °C for 14 hours. The reaction mixture was poured into 30 mℓ of ice-water and extracted twice with 20 mℓ portions of dichloromethane. The dichloromethane layer was separated, washed three times with 20 mℓ portions of water and then with 30 mℓ of a saturated sodium chloride aqueous solution, and dried over anhydrous sodium sulfate. The sodium sulfate was removed by filtration, and the resulting dichloromethane solution was concentrated under reduced pressure to obtain a yellowish brown oily substance. The substance was dried under reduced pressure overnight and purified by silica gel column chromatography using chloroform followed by a 30:1 (by volume) mixture of chloroform and methanol as developing solvents to obtain 132 mg of a 11-O-stearoyl derivative of Sf2587 substance and 39 mg of 3′,11-O-distearoyl derivative of SF2587 substance.
Properties of Compound 3: the compound of formula (I) wherein X = H; and Y = $CH_3(CH_2)_{16}CO$-
FDMS: m/Z 855 ($M^+$)

$$UV\ \lambda_{max}^{MeOH}\ nm\ (\varepsilon):\ 239\ (31,980),\ 260\ (29,970),\ 360\ (7,270)$$

Properties of Compound 4: the compound of formula (I) wherein X = $CH_3(CH_2)_{16}CO$-, Y = $CH_3(CH_2)_{16}CO$-
FDMS: m/Z 1121 ($M^+$)

$$UV\ \lambda_{max}^{MeOH}\ nm\ (\varepsilon):\ 240\ (23,820),\ 262\ (22,700),\ 363\ (5,380)$$

EXAMPLE 3

Synthesis of 3-O-Methanesulfonyl SF2587 Substance (Compound 5)

Four milliliters of pyridine were added to 10 mℓ of a dichloromethane solution containing 130 mg of SF2587 substance suspended therein and 170 μℓ of methanesulfonyl chloride was added thereto at room temperature while stirring. To the mixture was further added 7 mg of dimethylaminopyridine, followed by stirring at 25 °C for 24 hours. The reaction mixture was poured into 30 mℓ of ice-water and extracted twice with 20 mℓ portions of dichloromethane. The dichloromethane layer was separated, washed three times with 20 mℓ portions of water and then with 20 mℓ of a saturated sodium chloride aqueous solution, and dried over anhydrous sodium sulfate. The sodium sulfate was removed by filtration, and the resulting dichloromethane solution was concentrated under reduced pressure to obtain an orange oily substance. The substance was dried under reduced pressure overnight and purified by thin-layer chromatography using a silica gel plate and a 30:1 (by volume) mixture of toluene and acetone as a developing solvent to obtain 100 mg of an orange 3′-O-methanesulfonyl derivative of SF2587 substance.
Properties of Compound 5: the compound of formula (I) wherein X = $CH_3SO_3$-; and Y = H
FDMS: m/Z 667 ($M^+$)

UV $\lambda_{max}^{MeOH}$ nm (ε): 242 (54, 260), 265 (sh, 34,000), 410 (11,100), 423 (11, 270).

1H-NMR ($CDCl_3$, ppm): 13.28 s, 8.02 s, 7.89 d, 7.79 d, 6.44 s, 5.48 dd, 4.88 d, 4.42 dp, 3.34 d, 3.18 s, 3.12 dg, 2.94 s, 2.89 dd, 2.52 dd, 2.30 s, 1.92 s, 1.61 d, 1.50 dd, 1.44 d, 1.06 s.

## REFERENCE EXAMPLE

As a seed culture medium, a medium composed of 2.0% starch, 1.0% glucose, 0.6% wheat germ, 0.5% polypeptone, 0.3% yeast extract, 0.2% soybean meal, and 0.2% calcium carbonate was used.

As a production medium, a medium composed of 2.0% maltose syrup, 0.15% soybean oil, 1.0% soybean meal, 0.5% Pharmamedia, 0.25% distiller's soluble, 0.1% calcium carbonate, 0.0005% ferrous sulfate ($7H_2O$), 0.00005% cobalt chloride ($6H_2O$) and 0.0005% nickel chloride was ($6H_2O$) was used. Each medium was adjusted to pH 7.0 prior to sterilization.

A 100 mℓ Erlenmeyer flask containing 20 mℓ of the above seed culture medium was sterilized at 120°C for 30 minutes and then, inoculated with 2-3 loopfuls of Streptomyces sp. SF2587 (FERM BP-2244) grown on an agar slant. The incubation was performed under shaking at 28°C for 3 days to give a first seed culture. Then, a 500 mℓ Erlenmeyer flask containing 80 mℓ of the same seed culture medium as above was sterilized at 120°C for 30 minutes and, after cooling, inoculated with 2.4 mℓ of the above-prepared first seed culture. The incubation was carried out under shaking at 28°C for one day to give a second seed culture.

Four jar fermenters of 50-liter capacity, each containing 35 liters of the production medium, which was previously sterilized at 120°C for 30 minutes, were inoculated with 300 mℓ portions of said second seed culture. The incubation was carried out at 28°C for 4 days under aeration (20 ℓ/min.) and stirring (250 rpm).

After completion of incubation, the culture broth was filtered with the aid of diatomaceous earth to provide a cell-containing solid fraction.

This solid fraction was extracted with 60 ℓ of 67% acetone-water at 20°C with stirring and, then, filtered to remove the solid matter. This extract was distilled to remove acetone under reduced pressure and 10 ℓ of the resulting concentrate was extracted with 15 ℓ of ethyl acetate. The ethyl acetate layer was dehydrated over anhydrous sodium sulfate and concentrated under reduced pressure to give 8.98 g of oil. This oil was evenly mixed with 9 g of diatomaceous earth and dried under reduced pressure for 16 hours. It was then applied to a column in which 400 mℓ of silica gel C-200 (Wako Pure Chemical Industries) was packed with chloroform. The column was washed with chloroform and chloroform/methanol mixtures (100:1, 50:1, 20:1 and 10:1) in the order mentioned and finally elution was carried out with chloroform/methanol (5:1). The eluate was subjected to the cytotoxicity assay (MTT assay) against mouse leukemia cells (P-388) as described in Test Example 1 below and the fractions rich in cytotoxicity, which show 50% growth inhibition against P-388 cells when diluted 1:200, were pooled and concentrated to dryness under reduced pressure to provide 235 mg of oil. This oil was dissolved in a small amount of methanol and applied to a column in which 300 mℓ of Toyopearl HW-40 (Tosoh Corporation) was packed with methanol and elution was carried out with methanol. The eluate was subjected to the cytotoxicity assay using mouse leukemia P-388 cells and the active fractions, which show 100% growth inhibition against P-388 cells when diluted 1:122,000, were pooled, concentrated under reduced pressure and allowed to stand at 5°C for 16 hours. As a result, SF2587 substance separated out as a yellow precipitate. This precipitate was recovered by filtration and dried under reduced pressure at 40°C for 16 hours, whereby 3.0 mg purified SF2587 substance was obtained as a yellow powder.

## TEST EXAMPLE 1

### Cytotoxicity on P-388 Cells

Cytotoxicity of the compounds according to the present invention was determined by MTT assay in the following manner.

Mouse leukemia P-388 cells were suspended in RPMI medium supplemented with 10% fetal calf serum and this suspension was used to inoculate the 96-well microplate to give a cell concentration of $2 \times 10^3$ cells/well. The compound shown in Table 2 was dissolved in RPMI medium and add to each well for the test group, while no compound was added to wells of the control group. Incubation was carried out at 37° C for 72 hours at 5% $CO_2$. Then, MTT (3-(4,5-dimethylthiazol-2-yl)-2,5- diphenyl tetrazolium bromide) was added to each well to cause formation of formazan. The resulting formazan was dissolved by adding sodium dodecyl sulfate. Absorbance at 577-630 nm of each well was measured by the ELISA analyzer and 50% inhibitory concentration ($IC_{50}$) of each compound was calculated from the absorbance ratio of the test group to the control group. The results are shown in Table 2.

Table 2

| Compound No. | $IC_{50}$ (ng/mℓ) |
|---|---|
| 1 | 25.2 |
| 2 | 62.7 |
| 3 | 59.5 |
| 4 | 446.0 |
| 5 | 37.3 |

## TEST EXAMPLE 2

### Antitumor Activity

Antitumor activity of hydrochlorides of the compounds of the present invention was determined in the following manner.

Mouse leukemia P-388 cells maintained in the abdominal cavity of $BDF_1$ mouse were taken out and were suspended in physiological saline to give a cell concentration of $1 \times 10^6$ cells/0.2 mℓ. The cell suspension (0.2 mℓ) was transplanted to the abdominal cavity of 5-week- old male $BDF_1$ mice. The compounds of the present invention as shown in Table 3 was dissolved in 0.2 mℓ of physiological saline and was intraperitoneally administered to the mouse 24 hours after the transplantation of P-388 cells. No compound was administered to the mice of the control group. The T/C% value was calculated in accordance with the following formula.

$$T/C(\%) = \frac{\text{Survival days of mice of the test group}}{\text{Survival days of mice of the control group}} \times 100$$

The results are shown in Table 3.

Table 3

| Compound No. | Dose (mg/kg) | Anti-P388 Activity (T/C%) |
|---|---|---|
| 1 | 40.0 | 61.9 |
| 1 | 20.0 | 130.9 |
| 1 | 10.0 | 178.4 |
| 1 | 5.0 | 147.4 |
| 3 | 25.0 | 73.5 |
| 3 | 12.5 | 112.2 |
| 3 | 6.25 | 144.9 |
| 5 | 20.0 | 68.9 |
| 5 | 10.0 | 460.0 |
| 5 | 5.0 | 233.3 |

## TEST EXAMPLE 3

### Acute Toxicity

The hydrochlorides of the compounds of the present invention as shown in Table 4 were intraperitoneally administered to mice and acute toxicity ($LD_{50}$) of each compound was measured. The results are shown in Table 4.

Table 4

| Compound No. | $LD_{50}$ (mg/mℓ) |
|---|---|
| 1 | 20 |
| 3 | 15 |
| 5 | 15 |

As demonstrated above, the SF2587 substance derivative according to the present invention has reduced acute toxicity and enhanced antitumor activity as compared with the starting SF2587 substance and is thus promising as an anticancer agent.

## FORMULATION EXAMPLE 1

One gram of Compound 5 obtained in Example 3, an adequate amount of a peanut oil, and 1 g of benzyl alcohol were mixed, and a peanut oil was added thereof to make 100 mℓ. The solution was sealed in ampules by 1 me portions under sterile conditions.

## FORMULATION EXAMPLE 2

One gram of a hydrochloride of Compound 5 obtained in Example 3 was dissolved in 100 mℓ of

physiological salines. The solution was sterilized and sealed in ampules by 1 mℓ portions under sterile conditions.

FORMULATION EXAMPLE 3

Twenty gram of Compound 3 obtained in Example 2 and 65 g of polyethylene glycol (Macrogol 400) were mixed to form a uniform solution. Separately, a gelatin solution consisting of 45 g of gelatin, 10 g of glycerol, 5 g of D-sorbitol, 0.2 g of ethyl p-hydroxybenzoate, 0.1 g of propyl p-hydroxybenzoate, and 0.2 g of titanium oxide was prepared. Soft capsules each containing 200 mg of contents were produced using the above-prepared gelatin solution as a capsule-forming material according to a manual plate punching method.

## Claims

1. A compound represented by formula (I):

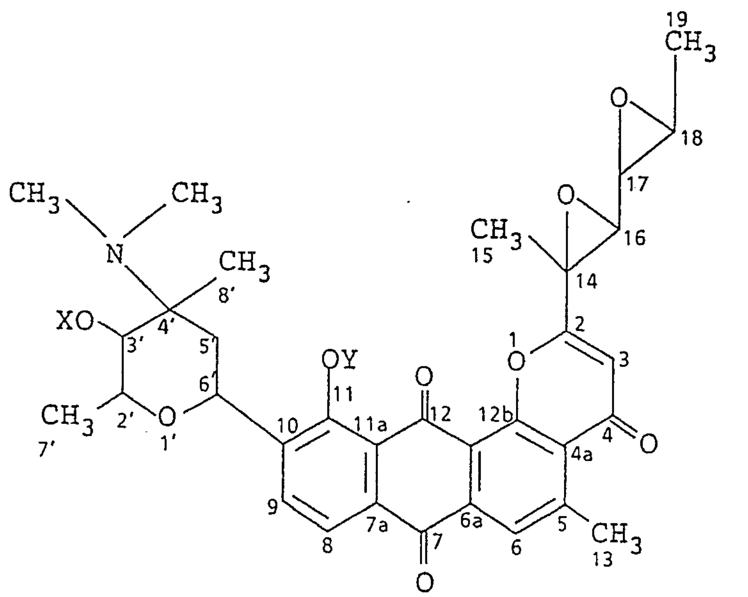

(I)

wherein X and Y represents a hydrogen atom, a group RCO-, wherein R represents an alkyl group having at least 5 carbon atoms or a substituted or unsubstituted aryl group, or a group $R'SO_2$-, wherein R′ represents a lower alkyl group or a substituted or unsubstituted aryl group provided that X and Y do not simultaneously represent a hydrogen atom, or a pharmaceutically acceptable salt thereof.

2. A compound of claim 1, wherein the alkyl group represented by R contains 5 to 19 carbon atoms.

3. A compound of claim 1, wherein the substituted aryl group represented by R is selected from a phenyl group, a p-hydroxyphenyl group, a p-tolyl group and p-bromophenyl group.

4. A compound of claim 1, wherein the lower alkyl group represented by R′ contains 1 to 5 carbon atoms.

5. A compound of claim 1, wherein the substituted aryl group represented by R′ is selected from a phenyl group, a p-tolyl group, a p-nitorophenyl group and a p-chlorophenyl group.

6. An anticancer composition containing a compound represented by formula (I)

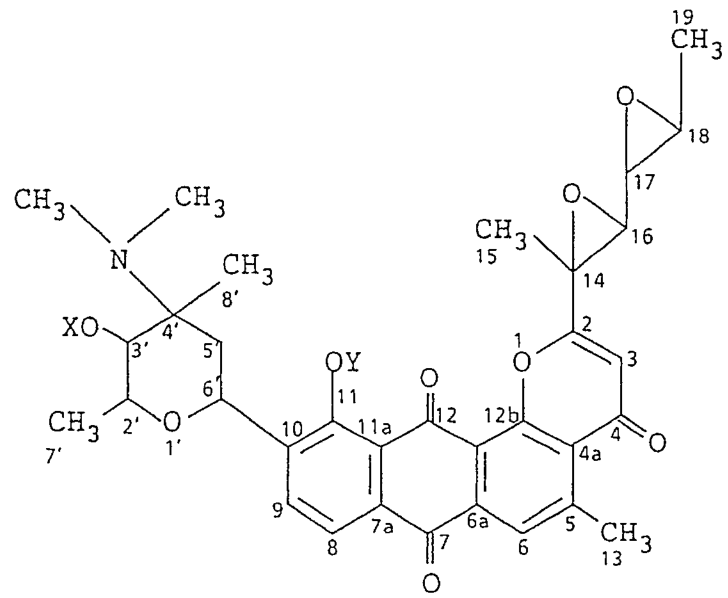

(I)

wherein X and Y represents a hydrogen atom, a group RCO-, wherein R represents an alkyl group having at least 5 carbon atoms or a substituted or unsubstituted aryl group, or a group $R'SO_2$-, wherein R′ represents a lower alkyl group or a substituted or unsubstituted aryl group provided that X and Y do not simultaneously represent a hydrogen atom, or a pharmaceutically acceptable salt thereof as an active ingredient, and a pharmaceutically acceptable carrier.

7. An anticancer composition of claim 6, wherein the alkyl group represented by R contains 5 to 19 carbon atoms.

8. An anticancer composition of claim 6, wherein the substituted aryl group represented by R is selected from a phenyl group, a p-hydroxyphenyl group, a p-tolyl group and p-bromophenyl group.

9. An anticancer composition of claim 6, wherein the lower alkyl group represented by R′ contains 1 to 5 carbon atoms.

10. An anticancer composition of claim 6, wherein the substituted aryl group represented by R′ is selected from a phenyl group, a p-tolyl group, a p-nitorophenyl group and a p-chlorophenyl group.

11. A process for preparing a compound of claim 1, which comprises reacting a compound represented by the formula (II)

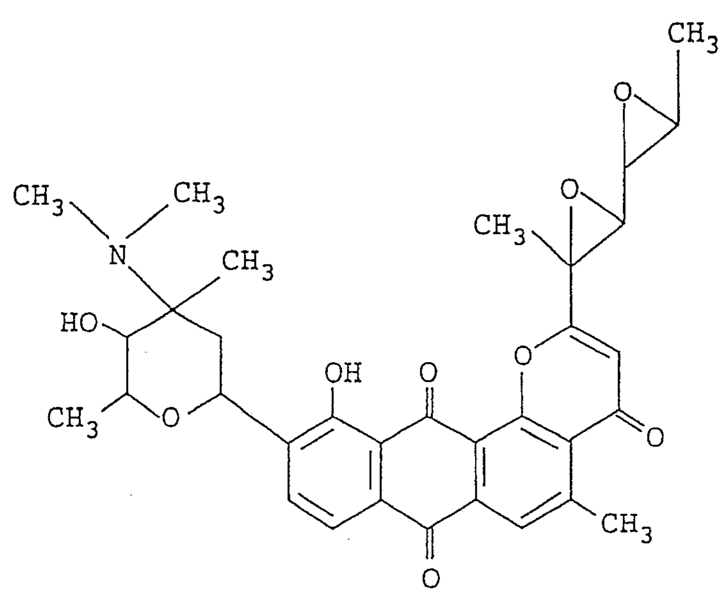

(II)

with an acylating agent or a sulfonylating agent in the presence of a base.

12. Use of a compound as claimed in claim 1 for the manufacture of a medicament for treating cancer.